# EUROPEAN PATENT SPECIFICATION

(11) **EP 2 068 818 B1**
(45) Date of publication and mention of the grant of the patent: **10.11.2010**
(21) Application number: 07820029.2
(22) Date of filing: 04.09.2007
(51) Int. Cl.: A61K 8/49, A61K 8/73, A61Q 19/00

(54) **COSMETIC COMPOSITIONS WITH ETHYLENE BRASSYLATE**
KOSMETISCHE ZUSAMMENSETZUNGEN MIT ETHYLENBRASSYLAT
COMPOSITIONS COSMÉTIQUES À BASE D'ÉTHYLÈNE BRASSYLATE

(30) Priority: 07.09.2006 US 470635
(43) Date of publication of application: 17.06.2009
(73) Proprietor: Unilever PLC, London EC4Y 0DY (GB); Unilever N.V., 3013 AL Rotterdam (NL)
(72) Inventor: OLSEN, Amy, Lynn, Monroe, Connecticut 06468 (US); MINER, Philip, Edward, Trumbull, Connecticut 06611 (US)
(74) Representative: Acham, Nicholas Clive
(86) International application number: PCT/EP2007/059220
(87) International publication number: WO 2008/028900

(56) References cited:
- WO-A-2005/107695
- US-A- 5 407 678
- US-B1- 6 555 097

## Description

### BACKGROUND OF THE INVENTION

### Field of the Invention

The invention concerns cosmetic compositions with aesthetics providing a silky afterfeel to the skin.

### The Related Art

Proper aesthetics are essential to any successful skin cream or lotion. Without the proper feel, consumers would not buy or use even those products with the most proven dermatological benefits.

Silkiness is known to be imparted by a variety of silicone materials. Yet the silicones do not transform compositions into a powdery afterfeel. Typical disclosures of silicone materials for cosmetics are found in US 5 972 359 (Sine et al.) and US 6 524 598 B2 (Sunkel et al.)

Starches have been employed to enhance viscosity of liquid formulations. For instance, US 5 824 323 (Fishman) reports skin lotion compositions with non-greasy skinfeel. These formulas can contain a variety of starches including tapioca to provide body and thickness to the lotions.

US 2006/0034876 A1 (Cheney et al.) reports on combinations of tapioca starch and polyacrylic beads which deliver a silky rub-in aesthetic transforming into a powdery drier skin afterfeel.

Although solutions have been suggested to solve the problem of delivering silky feel to the skin, there still remains the challenge of formulating a cosmetic that in a much greater variety of formulations can achieve the aesthetically required silky smooth skin afterfeel.

### SUMMARY OF THE INVENTION

A cosmetic composition is provided which includes:
(i) from 0.1 to 10% by weight of tapioca starch;
(ii) from 0.001 to 5% by weight of ethylene brassylate;
(iii) from 0.01 to 10% by weight of an emulsifier;
(iv) optionally, an effective amount to provide an initial silky feel upon skin contact of a polysiloxane material;
(v) optionally, from 0.1 to 40% by weight of polyhydric alcohol;
(vi) optionally, an effective amount to preserve of a preservative; and
(vii) a cosmetically acceptable carrier,
   wherein ethylene brassylate and tapioca starch are present in a relative weight ratio 1 :3 to 1 : 1.

### DETAILED DESCRIPTION OF THE INVENTION

Now there is provided a cosmetic composition of exceptionally pleasant aesthetics. The composition delivers a silky afterfeel to skin. The afterfeel aesthetics are achieved with a combination of tapioca starch and ethylene brassylate.

Tapioca starch is a first component of the present invention. Tapioca, also known as Cassava or Manioc, is a root or tuber extract. The plant is a perennial that grows eight to twelve feet high and the roots can be as much as three feet long and five to ten inches in diameter. Cells of the tuber carry the tapioca starch. This starch is recovered by wet grinding the washed roots and continuous re-washing, resulting in a pure carbohydrate. Particularly preferred is a powder source with a median particle size ranging from 1 to 100 micron (1000 nm to 100,000 nm), preferably from 5 to 15 micron, optimally from 10 to 30 micron.

The starch is commercially available under the tradename Tapioca Pure (28-1810) from the National Starch & Chemical Company, Division of ICI. An alternative source is TIStar Tapioca Starch sold by the Multi-Kem Corporation.

Amounts of tapioca starch may range from 0.1 to 10%, preferably from 0.5 to 5%, optimally from 0.3 to 1% by weight of the composition.

Advantageously the tapioca starch in certain formulations preferably is a water-insoluble partially hydrated granular substance having an average particle size ranging from 500 to 10,000 nm, preferably from 1,000 to 9,000 nm, optimally from 3,000 to 8,000 nm. Partially hydrated starches are achieved by heating tapioca starch in water to 50°C and holding this temperature for from 0.5 to 4 hours, preferably from 1 to 2 hours. Partial hydration converts from 10 to 80% by weight of the tapioca starch to a hydrated form.

Ethylene brassylate is a second component of the present invention. This material is available from many sources including Takasago International Corporation. Amounts of ethylene brassylate may range from 0.001 to 5%, preferably from 0.01 to 1%, and more preferably from 0.1 to 0.5% by weight of the composition.

Optionally the relative weight amounts of ethylene brassylate to tapioca starch may range from 1:5 to 1:2 by weight of the composition.

Emulsifiers are present in cosmetic compositions of the present invention. Total concentration of the emulsifier ranges from 0.01 to 10%, preferably from 0.1 to 5%, optimally from 1 to 3% by weight of the composition. The emulsifier may be selected from the group consisting of anionic, nonionic, cationic and amphoteric actives. Particularly preferred nonionic emulsifiers are those with a C₁₀-C₂₀ fatty alcohol or acid hydrophobe condensed with from 2 to 100 moles of ethylene oxide or propylene oxide per mole of hydrophobe; C₂₋C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic emulsifiers.

Polyhydric alcohols may be employed in certain compositions of the present invention. Typical polyhydric alcohols include glycerin (also known as glycerol), polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of polyhydric alcohol when present may range from 0.1 to 40%, preferably from 0.5 to 20%, optimally from 1 to 10% by weight of the composition.

Preservatives can desirably be incorporated into the cosmetic compositions of this invention to protect against the growth of potentially harmful microorganisms. Suitable traditional preservatives for compositions of this invention are alkyl esters of parahydroxybenzoic acid. Other preservatives which have more recently come into use include hydantoin derivatives, propionate salts, and a variety of quaternary ammonium compounds. Particularly preferred preservatives are phenoxyethanol, methyl paraben, propyl paraben, DMDM hydantoin, iodopropynyl butylcarbamate, 5-chloro-2-methyl-4-isothiazolin-3-one, 2-methyl-4-isothiazolin-3-one and benzyl alcohol. The preservatives should be selected having regard for the use of the composition and possible incompatibilities between the preservatives and other ingredients in the emulsion. Preservatives are preferably employed in amounts ranging from 0.00001% to 2% by weight of the composition.

Preferred anionic surfactants include soap, C₈-C₂₀ alkyl ether sulfates and sulfonates, C₈-C₂₀ alkyl sulfates and sulfonates, C₈-C₂₀ alkylbenzene sulfonates, C₈-C₂₀ alkyl and dialkyl sulfosuccinates, C₈-C₂₀ acyl isethionate, C₈-C₂₀ alkyl ether phosphates, C₈-C₂₀ sarcosinates, C₈-C₂₀ acyl lactylates and combinations thereof.

Polysiloxane materials may be present in compositions of this invention. The organopolysiloxane may be volatile, non-volatile, or a mixture of volatile and non-volatile silicones. The term "non-volatile" refers to those silicones that are liquid or solid under ambient conditions and have a flash point (under one atmosphere pressure) of at least about 100°C. The term "volatile" refers to all other silicone oils. Suitable organopolysiloxanes include polyalkylsiloxanes, cyclic polyalkylsiloxanes and polyalkylarylsiloxanes.

Polyalkylsiloxanes can be represented by the general chemical formula R₃SiO[R₂SiO]ₓSiR₃ wherein R is an alkyl group having from one to about 30 carbon atoms (preferably R is methyl or ethyl) and x is an integer from 0 to about 10,000, chosen to achieve the desired molecular weight which can range to over about 10,000,000. Commercially available polyalkylsiloxanes include the polydimethylsiloxanes, which are also known as dimethicones. These include the Vicas® series sold by General Electric Company and the Dow Corning® 200 series sold by Dow Coming Corporation. Dimethicones include those represented by the chemical formula (CH₃)₃SiO[(CH₃)₂SiO]ₓCH₃RSiO]_{y}Si(CH₃)₃ wherein R is straight or branched chain alkyl having from 2 to about 30 carbon atoms and x and y are each integers of 1 or greater selected to achieve the desired molecular weight which can range to over about 10,000,000. Examples of these alkylsubstituted dimethicones include cetyl dimethicone and lauryl dimethicone.

Cyclic polyalkylsiloxanes suitable for use in the composition include those represented by the chemical formula [SiR₂-O]ₙ wherein R is an alkyl group (preferably R is methyl or ethyl) and n is an integer from about 3 to about 8, more preferably from 4 to 6. Where R is methyl, these materials are typically referred to as cyclomethicones. Commercially available cyclomethicones include Dow Corning® 244 fluid which primarily contains the cyclomethicone tetramer (i.e. n=4), Dow Corning® 344 fluid which primarily contains the cyclomethicone pentamer (i.e. n=5), Dow Corning® 245 which primarily contains a mixture of the cyclomethicone tetramer and pentamer (i.e. n=4 and 5), and Dow Corning® 345 which primarily contains a mixture of the cyclomethicone tetramer, pentamer and hexamer (ie. n=4, 5 and 6).

Also useful are materials such as trimethylsiloxysilicate, which is a polymeric material corresponding to the general chemical formula [(CH₃)₃SiO_{1/2}]ₓ[SiO₂]_{y}, wherein x is an integer from about 1 to about 500 and y is an integer from about 1 to about 500. A commercially available trimethylsiloxysilicate is sold as a mixture with dimethicone as Dow Corning® 593 fluid.

Dimethiconols are also suitable for use in the composition. These compounds can be represented by the chemical formulas R₃SiO[R₂SiO]ₓSiR₂OH and HOR₂SiO[R₂SiO]ₓSiR₂OH wherein R is an alkyl group (preferably R is methyl or ethyl) and x is an integer from 0 to about 500, chosen to achieve the desired molecular weight. Commercially available dimethiconols are typically sold as mixtures with dimethicone or cyclomethicone (e.g. Dow Corning® 1401, 1402, 1403 and 1501 fluids). Particularly preferred is a blend with INCI name of Cyclopentasiloxane and PEG/PPG-20/15 Dimethicone commercially available from GE Silicones as SF1528.

Cross-linked organopolysiloxane elastomers may also be useful as polysiloxane materials. These may be of the emulsifying or non-emulsifying crosslinked elastomer variety. The term "non-emulsifying" defines a crosslinked organopolysiloxane elastomer from which polyoxyalkylene units are absent. The term "emulsifying" is used to mean crosslinked organopolysiloxane elastomer having at least one polyoxyalkylene unit.

Non-emulsifying silicone elastomers may be powders such as vinyl dimethicone/methicone silesquioxane crosspolymers available from Shin-Etsu as KSP-100, KSP-101, KSP-102, KSP-103, KSP-104 and KSP-105, hybrid silicone powders that contain a fluoroalkyl group such as KSP-200, and hybrid silicone powders that contain a phenyl group such as KSP-300; and Dow Corning material DC 9506.

Preferred organopolysiloxane compositions are dimethicone/vinyl dimethicone crosspolymers commercially available from Dow Coming (DC 9040 and DC 9045), General Electric (SFE 839), Shin Etsu (KSG-15, 16, 18 [dimethicone/phenyl vinyl dimethicone crosspolymer]), and Grant Industries (Gransil™ line of materials), and lauryl dimethicone/vinyl dimethicone crosspolymers supplied by Shin Etsu (KSG-31, KSG-32, KSG-41, KSG-42, KSG-43 and KSG-44).

Particularly useful emulsifying elastomers are polyoxyalkylene-modified elastomers formed from divinyl compounds, particularly siloxane polymers with at least two free vinyl groups, reacting with Si-H linkages on a polysiloxane backbone. Preferably, the elastomers are dimethyl polysiloxanes crosslinked by Si-H sites on a molecularly spherical MQ resin.

Combinations of emulsifying and non-emulsifying crosslinked siloxane elastomers may also be useful for purposes of this invention.

Amounts of the polysiloxane materials may range from 0.1 to 80%, preferably from 1 to 60%, optimally from 5 to 40% by weight of the composition.

Fatty acids having from 10 to 30 carbon atoms may in certain formulations also be suitable for compositions of the present invention. Illustrative of this category are pelargonic, lauric, myristic, palmitic, stearic, isostearic, hydroxystearic, oleic, linoleic, ricinoleic, arachidic, behenic and erucic acids. Particularly preferred is stearic acid. Amounts of the fatty acid may range from 0.1 to 20%, preferably from 0.5 to 10%, optimally from 1 to 5% by weight of the composition.

Another component which may be utilized in compositions of the present invention is that of polyacrylic beads. Especially useful are polymethyl methacrylate, polyethyl methacrylate, polyethyl acrylate and polymethyl acrylate polymers. Most preferred are polymethyl methacrylate beads. The beads can range in number average particle size from 1 to 50 micron, preferably from 3 to 30 micron, optimally from 5 to 10 micron. These beads may be cross-linked or non-cross-linked, but cross-linking is preferred. The beads may have an Oil Absorbance ranging from 60 to 300 ml/100g and preferably from 70 to 180 ml/100g, as defined by ASTM-D281-31. Beads of polymethyl methacrylate are sold under the trademark Ganzpearl, available from Presperse Incorporated (Piscataway, New Jersey 08854). Most preferred is Ganzpearl® GMP-0820 with number average particle size of about 8 micron and an Oil Absorbance of about 170 ml/100g.

Amounts of the polyacrylate beads may range from 0.001 to 5%, preferably from 0.01 to 1%, optimally from 0.1 to 0.5% by weight of the composition.

Compositions of this invention will include a cosmetically acceptable carrier. Amounts of the carrier may range from 1 to 99.9%, preferably from 50 to 95%, optimally from 80 to 90%. Among the useful carriers are water, emollients, fatty alcohols, thickeners and combinations thereof. The carrier may be aqueous, anhydrous or an emulsion. Preferably the compositions are aqueous, especially water and oil emulsions of the W/O or O/W variety. Water when present may be in amounts ranging from 1 to 95%, preferably from 20 to 70%, optimally from 35 to 60% by weight.

Emollient materials may serve as cosmetically acceptable carriers. These may be in the form of natural or synthetic esters and hydrocarbons. Amounts of the emollients may range anywhere from 0.1 to 95%, preferably between 1 and 50% by weight.

Among the ester emollients are:
a) Alkenyl or alkyl esters of fatty acids having 10 to 20 carbon atoms. Examples thereof include isoarachidyl neopentanoate, isononyl isonanonoate, oleyl myristate, isopropyl myristate, oleyl stearate and oleyl oleate.
b) Ether-esters such as fatty acid esters of ethoxylated fatty alcohols.
c) Polyhydric alcohol esters. Ethylene glycol mono- and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
d) Wax esters such as beeswax, spermaceti wax and tribehenin wax.
e) Sterols esters, of which cholesterol fatty acid esters are examples thereof.
f) Sugar esters of fatty acids such as sucrose polybehenate and sucrose polycottonseedate.
g) Natural esters useful in this invention are sunflower seed oil, safflower oil, cottonseed oil, olive oil, jojoba and mixtures thereof.

Hydrocarbons which are suitable cosmetically acceptable carriers include petrolatum, mineral oil, C₁₁-C₁₃ isoparaffins, polyalphaolefins, and especially isohexadecane, available commercially as Permethyl 101A from Presperse Incorporated.

Fatty alcohols having from 10 to 30 carbon atoms are another useful category of cosmetically acceptable carrier. Illustrative are stearyl alcohol, lauryl alcohol, myristyl alcohol and cetyl alcohol. Amounts may range from 0.05 to 20%, preferably from 0.1 to 2% by weight of the composition.

Thickeners can be utilized as part of the cosmetically acceptable carrier of compositions according to the present invention. Typical thickeners include polyacrylamides (e.g. Sepigel 305®), acryloyldimethyltaurate polymers and copolymers (e.g. Aristoflex AVC), cross-linked acrylates (e.g. Carbopol 982®), hydrophobically-modified acrylates (e.g. Carbopol 1382®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenum, pectin and combinations of these gums. Inorganics may also be utilized as thickeners, particularly clays such as bentonites and hectorites, fumed silicas, and silicates such as magnesium aluminum silicate (Veegum®). Amounts of the thickener may range from 0.0001 to 10%, usually from 0.001 to 1%, optimally from 0.01 to 0.5% by weight of the composition.

Cosmetic compositions of the present invention may be in any form. These forms may include lotions, creams, roll-on formulations, sticks, masks, mousses, aerosol and non-aerosol sprays and pad-applied (e.g. wipe) formulations.

Sunscreen actives may also be included in compositions of the present invention. Particularly preferred are such materials as ethylhexyl p-methoxycinnamate (available as Parsol MCX®), Avobenzene (available as Parsol 1789®) and benzophenone-3 (also known as Oxybenzone). Inorganic sunscreen actives may be employed such as microfine titanium dioxide and zinc oxide. Amounts of the sunscreen agents when present may generally range from 0.1 to 30%, preferably from 2 to 20%, optimally from 4 to 10% by weight.

Compositions of the present invention may also contain vitamins and their derivatives. By the term "derivatives" is meant C₁-C₄₀ alkyl esters, salts or isomers. The vitamins may include vitamin A and derivatives (e.g. retinyl palmitate, retinyl linoleate, retinyl acetate and retinoic acid), vitamin B (including niacinamide, DL-panthenol, pyridoxine palmitate, folic acid, carotin and biotin), vitamin C (including magnesium ascorbyl phosphate, ascorbyl tetraisopalmitate and ascorbyl glucoside), vitamin D, vitamin E (including tocopherol palmitate, tocopherol acetate and the alpha-, beta- and gamma- isomers of tocopherol) and combinations thereof. Total amount of vitamins when present in compositions according to the present invention may range from 0.00001 to 10%, preferably from 0.01% to 1%, optimally from 0.1 to 0.5% by weight of the composition.

Skin lightening agents may be included in the compositions of the invention. Illustrative substances are placental extract, lactic acid, niacinamide, arbutin, kojic acid, resorcinol and derivatives including 4-substitute resorcinols and combinations thereof. Amounts of these agents may range from 0.1 to 10%, preferably from 0.5 to 2% by weight of the composition.

Desquamation agents are further optional components. Illustrative are the alpha-hydroxycarboxylic acids and beta-hydroxycarboxylic acids. Among the former are salts of glycolic acid, lactic acid and malic acid. Salicylic acid is representative of the beta-hydroxycarboxylic acids. Amounts of these materials when present may range from 0.01 to 15% by weight of the composition.

A variety of herbal extracts may optionally be Included in compositions of this Invention. Illustrative are green tea, chamomile, licorice, lavender, grape seed and extract combinations thereof. The extracts may either be water soluble or water-insoluble carried in a solvent which respectively is hydrophilic or hydrophobic. Water and ethanol are the preferred extract solvents.

Flavonoids may also be included in compositions of this invention. The flavonoids may be selected from isoflavonoids, flavonols and combinations thereof. Particularly useful are glucosyl hesperidin and rutin. Amounts of these agents may range from 0.000001 to 5% by weight of the composition.

Anti-microbial agents may also be included in the compositions of this invention. Illustrative are trichlosan, trichlocarban, Octopyrox® and zinc pyrithione. Amounts may range from 0.01 to 5%, preferably from 0.1 to 0.5% by weight of the composition.

Colorants, fragrances, opacifiers and abrasives may also be included in compositions of the present invention. Each of these substances may range from 0.05 to 5%, preferably between 0.1 and 3% by weight of the composition.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

It should be noted that in specifying any range of concentration or amount, any particular upper concentration can be associated with any particular lower concentration or amount.

The following examples will more fully illustrate the embodiments of this invention. All parts, percentages and proportions referred to herein and in the appended claims are by weight unless otherwise illustrated.

### EXAMPLES 1-8

Typical body lotions according to the present invention are illustrated in examples 3 and 4 of table I. The remaining examples are comparative.

**TABLE I**

| | | EXAMPLE (WEIGHT %) | | | | | | | |
|---|---|---|---|---|---|---|---|---|---|
| TRADEMARK | CHEMICAL NAME | 1 | 2 | 3 | 4 | 5 | 6 | 7 | 8 |
| Pristerene 4911 | Stearic acid | 6.5 | 7.5 | 8.5 | 1.0 | 2.0 | 3.0 | 4.0 | 5.0 |
| Ritasynt | Glycerol monostearate/ Stearamide AMP | 1.5 | 1.5 | 1.5 | 1.5 | 1.5 | 1.5 | 1.5 | 1.5 |
| Kessco GMS | Glycerol monostearate | 0.5 | 1.0 | 0.5 | 0.5 | 1.0 | 0.5 | 1.5 | 1.5 |
| Cetyl Alcohol | Cetyl alcohol | 0.5 | 0.5 | 0.5 | 0.5 | 0.5 | 0.5 | 0.5 | 0.5 |
| IPM | Isopropyl myristate | 7.5 | 5.0 | 5.0 | 5.0 | 5.0 | 7.5 | 10.0 | 5.0 |
| Silicone Fluid 50 cts | Dimethicone | 4.0 | 5.0 | 6.0 | 2.0 | 2.0 | 4.0 | 4.0 | 8.0 |
| Disodium EDTA | Disodium EDTA | 0.05 | 0.05 | 0.05 | 0.05 | 0.05 | 0.05 | 0.05 | 0.05 |
| Glycerin | Glycerin | 5.0 | 5.0 | 5.0 | 5.0 | 5.0 | 5.0 | 10.0 | 10.0 |
| Veegum | Magnesium aluminum silicate | 0.3 | 0.3 | 0.3 | 0.3 | 0.3 | 0.3 | 0.3 | 0.3 |
| Keltrol CG 100 | Xanthan gum | 0.1 | 0.1 | 0.1 | 0.1 | 0.1 | 0.1 | 0.1 | 0.1 |
| Carbopol 934 (2% Active) | Carbomer | 9.0 | 9.0 | 9.0 | 12.0 | 12.0 | 6.5 | 6.5 | 6.5 |
| Titanium Dioxide | Titanium dioxide | 0.1 | 0.1 | 0.1 | 0.1 | 0.1 | 0.1 | 0.1 | 0.1 |
| Triethanolamine (99%) | Triethanolamine | 1.0 | 1.0 | 1.0 | 1.0 | 1.0 | 1.0 | 1.0 | 1.0 |
| DC 245 Fluid | Cyclopentasiloxane | 2.0 | 2.0 | 2.0 | 2.0 | 2.0 | 2.0 | 2.0 | 2.0 |
| DC 1501 | Dimethiconol | 0.5 | 0.5 | 0.5 | 0.5 | 0.5 | 0.5 | 0.5 | 0.5 |
| Ganzpearl GMP 0820 | Methyl methacrylate crosspolymer | 0.25 | 0.25 | 0.5 | 0.5 | 0.5 | 0.75 | 0.75 | 0.25 |
| GE SF 1528 | Dimethicone copolyol/ cyclomethicone | 0.75 | 1.0 | 0.75 | 0.75 | 2.0 | 2.0 | 5.0 | 0.25 |
| Tapioca Pure | Tapioca starch | 1.75 | 1.75 | 2.0 | 2.0 | 5.0 | 5.0 | 2.25 | 1.75 |
| Glydant Plus | Iodopropynyl butylcarbamate/DMDM hydantoin | 0.09 | 0.09 | 0.09 | 0.09 | 0.09 | 0.09 | 0.09 | 0.09 |
| Ethylene Brassylate | Ethylene brassylate | 0.1 | 0.5 | 1.0 | 2.0 | 1.0 | 1.5 | 0.8 | 0.3 |
| Fragrance | Fragrance | 0.15 | 0 | 1.0 | 0.75 | 0.75 | 0.15 | 0.15 | 1.1 |
| | Water | Balance | Balance | Balance | Balance | Balance | Balance | Balance | Balance |

The formulations of table I are blended in the following manner. Above specified quantities of water, disodium EDTA, titanium dioxide and gum xanthan/glycerin slurry (2%) are charged to a batching vessel. These components are mixed for five minutes and then the Carbomer is added as a solution. Heat is applied and the mixed contents are held at 80°C for fifteen minutes. The remaining glycerin is added. The oil phase ingredients are then charged to a separate vessel. Light mixing is begun and heat applied to achieve 80°C. Slowly, the oil phase is added to the water phase under moderate mixing while maintaining temperature. All remaining water is charged to the vessel. Then the contents are homogenized at 80°C for one minute. Contents are then cooled to 38-40°C accompanied by large sweep (75 rpm) mixing. Glydant Plus is added at 55°C. Cooling is continued accompanied by slow addition at no higher than 50°C of DC 1501, fragrance, ethylene brassylate and tapioca along with the various minor ingredients.

### EXAMPLE 9

Another oil-in-water emulsion according to the present invention is described under table II.

**TABLE II**

| | INGREDIENT | WEIGHT % |
|---|---|---|
| Phase A | | |
| | Distilled water | balance |
| Phase B | | |
| | Glycerin | 5.00 |
| | Titanium dioxide | 0.75 |
| Phase C | | |
| | Glycerin | 1.00 |
| | Disodium EDTA | 0.10 |
| | Carbopol® 954 | 0.68 |
| | Carbopol® 1382 | 0.10 |
| | Tapioca starch | 2.50 |
| Phase D | | |
| | Cetyl alcohol | 0.70 |
| | Stearyl alcohol | 0.50 |
| | Stearic acid | 0.10 |
| | PEG-100 stearate | 0.10 |
| | Glycerol monostearate | 1.00 |
| | Dimethicone | 2.00 |
| | Cyclomethicone | 0.65 |
| | Tocopherol acetate | 0.10 |
| | Niacinamide | 2.00 |
| Phase E | | |
| | Distilled water | 2.00 |
| | NaOH | To neutralize |
| Phase F | | |
| | Urea | 2.00 |
| | D-Panthenol | 0.10 |
| | Distilled water | 5.00 |
| Phase G | | |
| | Glydant Plus | 0.10 |
| | Glycerin | 1.00 |
| | Distilled water | 1.00 |
| Phase H | | |
| | Ethylene brassylate | 1.00 |
| | Methyl isostearate | 1.50 |
| | Ganzpearl® GMP 0820 | 0.50 |
| | Retinol | 0.50 |
| | Butylated hydroxy toluene | 0.05 |
| | Tween® 20 | 1.00 |

### EXAMPLE 10

A hand cream according to the present invention is herein reported under table III.

**TABLE III**

| INGREDIENT | WEIGHT % |
|---|---|
| Distearyldimonium chloride (Varlsoft TA100®) | 4.1 |
| Cetyl alcohol | 2.9 |
| Steareth 21 | 1.7 |
| Stearyl stearate | 0.01 |
| Petrolatum | 0.3 |
| Dimethicone 200/200 | 1.0 |
| Mineral Oil 1000SUS | 2.8 |
| Isopropyl palmitate | 4.0 |
| Soy sterol | 0.1 |
| Stearic acid | 0.1 |
| Lecithin | 0.1 |
| Borage seed oil | 0.1 |
| Vitamin E acetate | 0.1 |
| Propylparaben | 0.1 |
| Sodium chloride | 0.2 |
| Glycerin | 10.0 |
| Titanium dioxide | 0.1 |
| Methylparaben | 0.2 |
| Tapioca starch | 0.5 |
| DMDM hydantoin | 0.3 |
| Ethylene brassylate | 0.2 |
| Water | Balance |

### EXAMPLE 11

A series of experiments were conducted to evaluate skinfeel performance through instrumentation. Various combinations of tapioca starch and ethylene brassylate were evaluated for slip friction as a function of time. This procedure measures friction during the first ten minutes of drying after application. The test is conducted in an environmentally controlled chamber at 21 °C and 20% relative humidity. Sample size of 100 microliters is spread on a Lucite table over a 6 by 5 inch area (2.54 cm to the inch). The table is attached to the cross-head of an Instron Model 4501 Materials Testing System. A 3 by 1 inch aluminum sled covered with a 100% rayon non-woven is pulled across the same area at a rate of 10 cm/min, starting one minute after application and repeated each minute for ten minutes. The integral of force vs. a distance of 50 mm (i.e. amount of work, units of gram-mm) is calculated for each of the intervals. Three runs are made of each product.
Table IV identifies the amounts of tapioca and ethylene brassylate for each sample In a base formula similar to that of example 1 (water varied in accordance with levels of tapioca starch, ethylene brassylate and corn starch). Slip friction results are reported in table V.

**TABLE IV**

| Sample | Tapioca (weight %) | Ethylene Brassylate (weight %) | Com Starch (weight %) |
|---|---|---|---|
| A | 0 | 2.0 | 0 |
| B | 0.5 | 1.5 | 0 |
| C | 2.0 | 0 | 0 |
| D | 1.0 | 1.0 | 0 |
| E | 1.5 | 0.5 | 0 |
| F | 0 | 1.0 | 1.0 |

**TABLE V**

| Sample | Slip Work Load (gram-mm) |
|---|---|
| A | 768.2 |
| B | 743.8 |
| C | 742.3 |
| D | 662.9 |
| E | 633.1 |
| F | 728.5 |

The results shown in table V demonstrate that a combination of ethylene brassylate and tapioca starch (samples D and E) provide better slip results (lower friction) than either of the separate materials (samples A and C). Further, the results of ethylene brassylate with tapioca are much better than the ethylene brassylate combination with corn starch. Compare sample C to sample F.

## Claims

1. A cosmetic composition comprising:
(I) from 0.1 to 10% by weight of tapioca starch;
(II) from 0.001 to 5% by weight of ethylene brassylate;
(III) from 0.01 to 10% by weight of an emulsifier;
(iv) optionally, an effective amount to provide an initial silky feel upon skin contact of a polysiloxane material;
(v) optionally, from 0.1 to 40% by weight of polyhydric alcohol;
(vi) optionally, an effective amount to preserve of a preservative; and
(vii) a cosmetically acceptable carrier. wherein ethylene brassylate and tapioca starch are present in a relative weight ratio from 1:3 to 1:1.

2. A cosmetic composition according to claim 1, wherein the tapioca starch is present in an amount from 0.5 to 5% by weight of the composition.

3. A cosmetic composition according to claim 1, wherein ethylene brassylate is present in an amount from 0.1 to 0.5% by weight of the composition.

4. A cosmetic composition according to claim 1, wherein the tapioca starch is a partially hydrolyzed material.

5. A cosmetic composition according to claim 1, wherein the polyhydric alcohol is glycerin.

6. A cosmetic composition according to claim 1, wherein the polysiloxane material is present from 0.1 to 80% by weight of the composition.

## Patentansprüche

1. Kosmetische Zusammensetzung, umfassend:
(i) von 0,1 bis 10 Gewichts-% Tapiokastärke;
(ii) von 0,001 bis 5 Gewichts-% Ethylenbrassylat;
(iii) von 0,01 bis 10 Gewichts-% eines Emulgators;
(iv) gegebenenfalls eine wirksame Menge, um ein anfängliches seidiges Gefühl bei Hautkontakt bereitzustellen, eines Polysiloxan-Materials;
(v) gegebenenfalls von 0,1 bis 40 Gewichts-% mehrwertiger Alkohol;
(vi) gegebenenfalls eine zur Konservierung wirksame Menge eines Konservierungsmittels und
(vii) einen kosmetisch verträglichen Träger,
wobei Ethylenbrassylat und Tapiokastärke in einem relativen Gewichtsverhältnis von 1:3 bis 1:1 vorliegen.

2. Kosmetische Zusammensetzung nach Anspruch 1, wobei die Tapiokastärke in einer Menge von 0,5 bis 5 Gewichts-% der Zusammensetzung vorliegt.

3. Kosmetische Zusammensetzung nach Anspruch 1, wobei Ethylenbrassylat in einer Menge von 0,1 bis 0,5 Gewichts-% der Zusammensetzung vorliegt.

4. Kosmetische Zusammensetzung nach Anspruch 1, wobei die Tapiokastärke ein partiell hydrolysiertes Material ist.

5. Kosmetische Zusammensetzung nach Anspruch 1, wobei der mehrwertige Alkohol Glycerin ist.

6. Kosmetische Zusammensetzung nach Anspruch 1, wobei das Polysiloxan-Material in einer Menge von 0,1 bis 80 Gewichts-% der Zusammensetzung vorliegt.

## Revendications

1. Composition cosmétique comprenant :
(i) de 0,1 à 10 % en poids d'amidon de tapioca ;
(ii) de 0,001 à 5 % en poids de brassylate d'éthylène ;
(iii) de 0,01 à 10 % en poids d'un émulsifiant ;
(iv) éventuellement, une quantité efficace pour obtenir une sensation soyeuse initiale au contact de la peau d'un matériau de polysiloxane ;
(v) éventuellement, de 0,1 à 40 % en poids de polyol ;
(vi) éventuellement, une quantité efficace pour conserver d'un conservateur ; et
(vii) un véhicule acceptable sur le plan cosmétique ;
dans laquelle le brassylate d'éthylène et l'amidon de tapioca sont présents dans un rapport en poids relatif de 1/3 à 1/1.

2. Composition cosmétique selon la revendication 1, dans laquelle l'amidon de tapioca est présent en une quantité de 0,5 à 5 % en poids de la composition.

3. Composition cosmétique selon la revendication 1, dans laquelle le brassylate d'éthylène est présent en une quantité de 0,1 à 0,5 % en poids de la composition.

4. Composition cosmétique selon la revendication 1, dans laquelle l'amidon de tapioca est un matériau partiellement hydrolysé.

5. Composition cosmétique selon la revendication 1, dans laquelle le polyol est le glycérol.

6. Composition cosmétique selon la revendication 1, dans laquelle le matériau de polysiloxane est présent en une quantité de 0,1 à 80 % en poids de la composition.
